Europäisches Patentamt

European Patent Office

Office européen des brevets

(11) Publication number: **0 306 977 B1**

# EUROPEAN PATENT SPECIFICATION

(45) Date of publication of patent specification: **12.01.94**  (51) Int. Cl.5: **G01N 33/52,** //G01N35/00

(21) Application number: **88114793.8**

(22) Date of filing: **09.09.88**

The file contains technical information submitted after the application was filed and not included in this specification

(54) **Method for quantification of a specific component deposited on a chemical analysis tape.**

(30) Priority: **11.09.87 JP 228036/87**

(43) Date of publication of application:
**15.03.89 Bulletin 89/11**

(45) Publication of the grant of the patent:
**12.01.94 Bulletin 94/02**

(84) Designated Contracting States:
**DE GB**

(56) References cited:
**GB-A- 1 218 749**
**SU-A- 390 436**
**US-A- 3 261 668**
**US-A- 3 992 158**

**DERWENT ABSTRACTS of SU A
390436&NUM;**

(73) Proprietor: **Fuji Photo Film Co., Ltd.**
**210 Nakanuma**
**Minamiashigara-shi**
**Kanagawa-ken(JP)**

(72) Inventor: **Saito, Yoshio, Fuji Photo Film Co.
Ltd.**
**3-11-46 Senzui**
**Asaka-shi, Saitama-ken(JP)**
Inventor: **Ishizuka, Kimiko, Fuji Photo Film Co.
Ltd.**
**3-11-46 Senzui**
**Asaka-shi, Saitama-ken(JP)**

(74) Representative: **Patentanwälte Grünecker,
Kinkeldey, Stockmair & Partner**
**Maximilianstrasse 58**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 306 977 B1

# Description

## BACKGROUND OF THE INVENTION

### Field of the Invention

This invention relates to a method for quantification of a specific component, deposited on a chemical analysis tape, said specific component being contained in a sample liquid, e.g., body fluid such as blood, serum, urine or the like.

### Description of the Prior Art

Quantitative analysis of various metabolic components in body fluid, e.g., glucose, bilirubin, urea nitrogen, uric acid, cholesterol, lactate dehydrogenase, creatine kinase, GOT, GPT, is important in clinical medicine and is essential for diagnosis, follow-up of the results of treatment, determination of prognosis and the like. In a clinical chemical examination using blood or the like as a sample, it is preferred that an accurate result can be obtained with a very small amount of sample liquid. Conventionally, a wet method using a reagent solution is employed, but it takes a relatively long time.

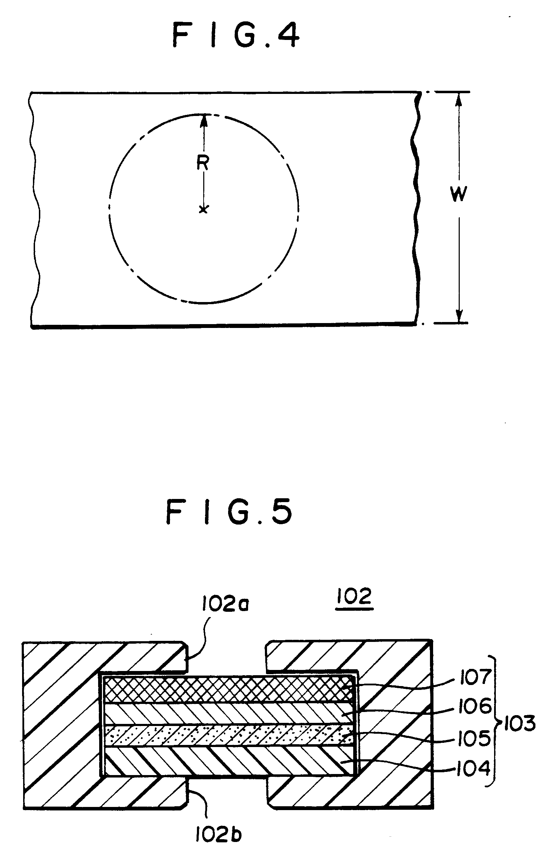

In order to quickly performing such an examination, there has been developed a chemical analysis slide comprising a dry type multiple layer film. Though the chemical analysis slide can be divided into a plurality of types depending on the structure of the multiple layer film, typically it comprises, as shown in Figure 5, a plastic slide frame 102 having a sample liquid depositing opening 102a through which a drop of sample liquid is deposited and a reflection density measuring opening 102b, and a dry type multiple layer film 103 received in the slide frame 102. The dry type multiple layer film 103 comprises a transparent base film 104, a reagent layer 105, a light reflecting layer 106 and a spreading layer 107. The base film 104 may be of a thin primed plastic film, for example. In the reagent layer 105 is contained a reagent which reacts with the component-to-be-analyzed in the sample liquid and is colored into an optical density according to the amount of the component-to-be-analyzed. The light reflecting layer 106 prevents light entering the reagent layer 105 from reaching the spreading layer 107 so that the sample liquid deposited on the reagent layer 107 affects measurement of the optical density. The spreading layer 107 uniformly spreads the sample liquid deposited thereon into an area substantially proportional to the amount of the sample liquid. When performing quantitative analysis by the use of the chemical analysis slide, a predetermined amount of sample liquid, e.g., whole blood, is deposited on the surface of the spreading layer 107 through the sample liquid depositing opening 102a. The sample liquid spread by the spreading layer 107 reaches the reagent layer 105 through the light reflecting layer 106 and reacts with the reagent in the reagent layer 105 to be colored. After deposition of the sample liquid, the chemical analysis slide is incubated at a predetermined temperature for a predetermined time to promote color reaction. Thereafter, measuring light is projected onto the reagent layer 105 through the optical density measuring opening 102b, and reflecting light in a specific wave range is measured, thereby obtaining the optical density. Then, the component-to-be-analyzied is quantified on the basis of the optical density with reference to a calibration curve set in advance.

The chemical analysis slide is economically disadvantageous in the following points.

First the chemical analysis slide costs much labor to produce. Said chemical analysis film is formed by cutting a large-area chemical analysis film into numbers of small film chips, and each film chip is fixed to a slide frame. Especially, the process of cutting the film chips with correct dimensions, fixing the chip to the frame in the correct position and incorporating parts of the frame lots of labor. Though labor can be saved by using an automated machine, it costs much initial investment and labor for maintenance of the machine. Second the material of the frame is expensive and processing of the frame costs much.

By the use of a chemical analysis tape, i.e., a chemical analysis film in a continuous length instead of such a slide, various costs described above can be saved.

Such chemical analysis tapes are disclosed in United States Patent Nos. 3,260,413; 3,526,480; and 3,992,158, for instance. However, in the tape disclosed in the former two United States patents, two or more tapes are temporarily brought into contact with each other to form a composite structure and subsequently separated from each other, and accordingly, the chemical analyzing apparatus using the tape must be complicated in structure. On the other hand, the tape disclosed in the last United States patent is in the form of an integrated multiple layer chemical analysis tape and accordingly, the chemical analyzing apparatus using the tape may by relatively simple in structure. However, the integrated multiple layer chemical analysis tape is still economically disadvantageous in that a substantially large part of the tape is not available for analysis and is wasted. Since the tape contains all reagents necessary for analysis and cost of the tape per unit area is very expensive, existence of such an idle part substantially adds to the costs of chemical examination. That is, in the integrated multiple layer chemical analysis tape disclosed in Untied States Patent No. 3,992,158, the sample liquid deposited thereon is uniformly spread in all the directions by an outermost liquid spreading layer, and accordingly, the sample liquid is spread in a circular region as shown by chained line in Figure 4. In the case where the amount of the sample liquid is 5 to 10 $\mu\ell$, which is normal, the diameter of the circular region is about 10mm. Accordingly, the width of the tape must be larger than 10mm.

In the chemical analysis tape, the part available for analysis is the part in which the sample liquid is spread and in which a detectable change such as coloring occurs. When the radius of the circular region in which the sample liquid is spread is represented by R(mm), the distance between adjacent sample spots is represented by B(mm) and the width of the tape is represented by W(mm), the area A of the circular region in which the sample liquid is spread is equal to $\pi R^2 mm^2$ and the area of each analyzing unit is equal to W(2R + B). Accordingly, the area of the idle part is equal to W-(2R + B)- $\pi R^2$. The proportion of the area of the idle part to the whole area of each analyzing unit is expressed as follows.

$$1-[\pi R^2/W(2R + B)]$$

Since B is generally equal to W-2R, the proportion is expressed as follows.

$$1- \pi(R^2/W^2) = 1- \pi(R/W)^2$$

When it is assumed that 2R/W is 0.7, the proportion of the area of the idle part to the whole area of each analyzing unit becomes as follows.

$$1- \pi(R-W)^2 = 1- \pi(7/20)^2 = 0.62$$

That is, 62% of the whole area of each analyzing unit is wasted.

A method for quantification of a specific component, deposited on a chemical analysis tape according to the preamble of the claim is disclosed in SU-A-390 436. It is proposed in this document to move the tape in regular intervals by steps, with a pitch which is smaller than the width of the produced color spots. To produce clear marking, the pitch is reduced in invert proportion to the concentration of the tested substance. If the sample is spread on the tape in the form of a circular area, the continuing spots will affect each other, thereby preventing a clear read-out of the color.

In view of the foregoing observations and description, the primary object of the present invention is to provide a method for quantification of a specific component, deposited on a chemical analysis tape in which the proportion of the idle part is minimized.

The method in accordance with the present invention is characterized in that the width of the chemical analysis tape satisfies formula $\sqrt{2S/3P\pi} <$ W $< 2\sqrt{S/P\pi}$ wherein S represents in $\mu\ell$ the volume of the sample liquid to be deposited on the tape, P represents in $\mu\ell$ the quantity of the sample liquid which the spreading layer receives per unit area ($mm^2$) thereof, and W represents the width of the tape in mm.

When W $> 2\sqrt{S/P\pi}$, liquid can be freely spread in all the directions on the chemical analysis tape.

In the case of the aforesaid chemical analysis slide or a chemical analysis tape the width of which is sufficiently large as compared with the area of the spreading region (The region over which the sample liquid is spread.), the sample liquid is uniformly spread in all the directions and accordingly, it can be expected that the sample liquid is spread with direct proportional relation between the quantity of the sample liquid and the area of the spreading region being maintained. However, in the case of a chemical analysis tape the width of which is smaller than the diameter of the free spreading region (The aforesaid circular region in which the sample liquid is spread when the sample liquid is freely spread.), spread of the liquid in the transverse direction of the tape is limited and accordingly, it cannot be expected that the area of the spreading region is in proportion to the quantity of the sample liquid. However, so long as the volume S of the sample liquid to be deposited on the tape, the quantity P of the sample liquid which the spreading layer receives per unit area thereof, and the width W of the tape satisfy the formula $\sqrt{2S/3P\pi} < W < 2\sqrt{S/P\pi}$, the area of the spreading region can be substantially in proportion to the quantity of the sample liquid even when the width of the tape is smaller than the diameter of the free spreading region. By making the width of the tape smaller than the diameter of the free spreading region, the proportion of the area of the idle part to the whole area of each analyzing unit can be substantially reduced.

In order to further reduce the proportion of the area of the idle part, it is preferred that the following formula is satisfied.

$$\sqrt{2S/3P\pi} < W < \sqrt{3S/P\pi}$$

BRIEF DESCRIPTION OF THE DRAWINGS

Figure 1 is a plan view showing a chemical analysis film in accordance with the present invention,

Figure 2 is a cross-sectional view showing an example of the layer arrangement of the chemical analysis tape,

Figure 3 is a schematic view showing the chemical analyzing apparatus using the chemical analysis tape,

Figure 4 is a plan view for illustrating the conventional chemical analysis tape, and

Figure 5 is a cross-sectional view for illustrating the conventional chemical analysis slide.

## DETAILED DESCRIPTION OF THE INVENTION

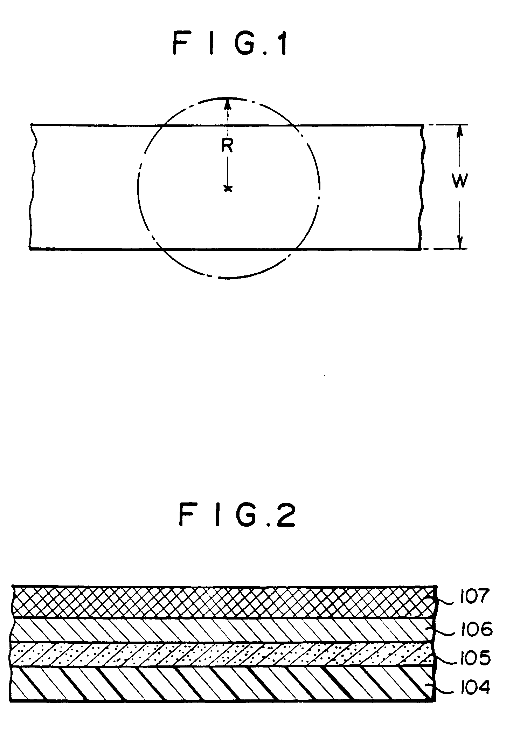

As shown in Figure 1, in accordance with the present invention, the width W of the tape is selected to be smaller than the diameter 2*R of the free spreading region shown by the chain line in Figure 1. The layer arrangement of the tape may be the same as that of the chemical analysis tape 103 described above in conjunction with Figure 5, and an example of the layer arrangement of the tape is shown in Figure 2.

As the chemical structure (layer arrangement) of the chemical analysis tape of the present invention, those disclosed in Japanese Unexamined Patent Publication Nos. 55(1980)-164,356; 59(1984)-102,388; and 60(1985)-222,769; United States Patent 3,992,158 and the like can be employed in addition to that shown in Figure 2.

When a water-impermeable and light-transmitting film is employed as the base film, the chemical analysis film of the present invention may have one of the following layer arrangements, for example.

(1) A spreading layer containing a reagent composition and formed on the base film.

(2) A reagent layer and a spreading layer formed on the base film in this order.

(3) A detecting layer, a reagent layer, and a spreading layer formed on the base film in this order.

(4) A reagent layer, a light reflecting layer, and a spreading layer formed on the base film in this order.

(5) A detecting layer, a reagent layer, a light reflecting layer and a spreading layer formed on the base film in this order.

(6) A detecting layer, a light reflecting layer, a reagent layer and a spreading layer formed on the base film in this order.

A water absorbing layer may be inserted between the base film and the spreading layer or between the base film and the reagent layer in the layer arrangements of (1), (2) and (4). A filtering layer may be inserted between the reagent layer and the detecting layer or between the reagent layer and the spreading layer in the layer arrangements of (2) and (5). A blood cell filtering layer and/or an inhibitor removing layer may be inserted between the light reflecting layer and the detecting layer, between the light reflecting layer and the reagent layer, between the light reflecting layer and the spreading layer or between the reagent layer and the spreading layer. The detecting layer is a layer in which the pigment or the like formed under the existence of the component-to-be-analyzed diffuses and is optically detected through the base film, and may be formed of a hydrophilic polymer. The water absorbing layer is a layer in which the pigment formed under the existence of the component-to-be-analyzed does not substantially diffuse and may be formed of a hydrophilic polymer which is apt to swell.

Preferably the spreading layer is adapted to deliver the deposited sample liquid to the adjacent water-permeable layer so that the water-permeable layer receives substantially uniform quantity of the sample liquid per unit area thereof. For example, the spreading layer may be of fiber material such as fabric disclosed in United States Patent No. 4,292,272 or knit disclosed in Japanese Unexamined Patent Publication No. 60(1985)-222,769. The fabric and the like may be processed by glow discharge as disclosed in Japanese Unexamined Patent Publication No. 57(1982)-66,359. The spreading layer may be provided with hydrophilic polymer or surface-active agent in order to control the spreading area, spreading speed and the like as disclosed in Japanese Unexamined Patent Publication Nos. 60(1985)-222,770; 61(1986)-122,875; 61(1986)-122,876; and 61(1986)-143,754, for example.

The spreading layer may be bonded to the underlying layer, i.e., the reagent layer, the light reflecting layer, the filtering layer, the water absorbing layer, or the detecting layer, by an adhesive layer. The adhesive layer may be of hydrophilic polymer such as gelatin, gelatin derivative, polyacrylamide, or starch which can bond a porous layer when it is swelled with water.

As described above, the chemical analysis tape of the present invention may be provided with a light reflecting layer. The light reflecting layer conceals the color of the sample liquid deposited on the spreading layer, e.g., red of hemoglobin or yellow of bilirubin, when optically detecting detectable changes (e.g., change in color, coloring and the like) occurring in the detecting layer and/or the reagent layer through the light transmitting base film, and reflects light projected through the base film. Preferably the light reflecting layer is a water-permeable layer in which light reflecting fine particles such as of titanium oxide, barium sulfate or the like are dispersed.

In the reagent layer and the like, there is contained optically detectable matter, for instance, a composition which can form a dye. For example, compositions which can form a dye through oxidation of leuco-dye (e.g., arylimidazole leuco-dye disclosed in United States Patent No. 4,089,747 and Japanese Unexamined Patent Publication No. 59-(1984)-193,352), compositions containing a compound which forms a dye through coupling with other compound(s) when it is oxidized (e.g., 4-aminoantipyrine, phenols, and naphthols) and compositions comprising a compound which can form a dye under the existence of a reducing coenzyme and an electron transport agent can be used. In the case of the chemical analysis tape for measuring enzyme activity, the reagent layer and/or the spreading layer may contain a self-developing substrate which can liberate a colored material such as p-nitrophenol. Preferably the reagent layer is a substantially uniform layer containing hydrophilic polymer as binder.

It is preferred that the chemical analyzer using the chemical analysis tape in accordance with the present invention be provided with an automatic dispenser which can deposit sample liquid on the chemical analysis tape in a controlled amount. When the width W (mm) of the chemical analysis tape, the quantity P ($\mu\ell$) of the sample liquid which the spreading layer receives per unit area thereof and the quantity S ($\mu\ell$) of the sample liquid deposited on the chemical analysis tape satisfy the following formula, the area of the spreading region and the quantity of the sample liquid deposited on the chemical analysis tape can be substantially in proportion to each other.

$$1/4(\pi PW^2) < S < 3/2(\pi PW^2)$$

When the radius of the spreading region as measured in the longitudinal direction of the tape is represented by R(mm), the distance between the analyzing units is represented by B(mm) and the width of the tape is represented by W(mm), the area A (mm$^2$) of the spreading area is as represented by the following formula.

$$A = 2R^2\{sin^{-1}(W/2R)\} + W \sqrt{R^2-(W/2)^2}$$

For example, when R = $\sqrt{2}$ (W/2),

$$A = \pi R^2/2 + W^2/2$$
$$= (\pi/2 + 1)W^2/2$$

Since the area (mm$^2$) of each analyzing unit is represented by formula W(2R + B) = 2RW + WB, the area of the idle part to be wasted is represented by the following formula.

$$2RW + WB - A$$
$$= \sqrt{2} W^2 + WB - (\pi/2 + 1)W^2/2$$

$$= W^2\{\sqrt{2} - 1/2(\pi/2 + 1)\} + WB$$
$$= W^2(1.41 - 1.28) + WB$$
$$= W(0.13W + B)$$

The proportion of the area of the idle part to the whole area of each analyzing unit is represented by formula

$$0.13W + B: \sqrt{2}W + B$$

When B is equal to W/2,
W(0.13 + 0.5):W(1.41 + 0.5) = 0.63:1.91 = 0.33:1.
Thus, in this case, the idle part occupies only 33% of the whole area of each analyzing unit.

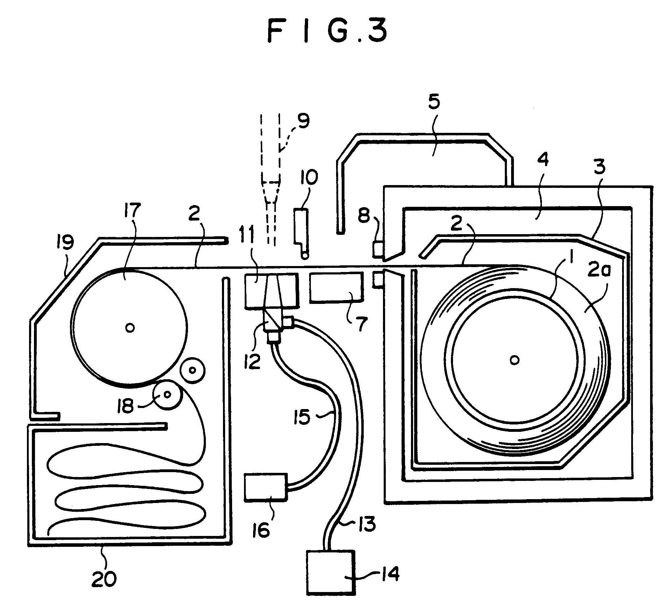

Figure 3 shows an example of an automatic chemical analyzing apparatus in which the chemical analysis tape of the present invention can be suitably used. In Figure 3, a chemical analysis tape 2 in accordance with the present invention is wound around a reel 1 into a roll 2a and is accommodated in a cassette 3. The cassette 3 is accommodated in an insulating container 4 having a window provided with a shutter 8. The chemical analysis tape 2 is drawn from the cassette 3 and outside the insulating container 4 through the window. Low humidity air is blown over the upper surface of the tape 2 by an air blower 5, and the lower surface of the tape is preheated to an appropriate temperature near the room temperature by a preheater 7. Thereafter, the tape 2 is brought to a reflection density measuring section comprising a sample liquid depositing means 9, a temperature maintaining means 11 and an optical measuring probe 12. The sample liquid depositing means 9 may comprise a micro pipette, dispenser or the like. The temperature maintaining means 11 may be a block kept at a predetermined temperature by an electric heater. The optical measuring probe 12 projects a measuring light beam onto the lower surface of the tape 2 and receives reflected light therefrom. The quantity of the reflected light is measured and the reflection density of the surface exposed to the measuring light beam is determined on the basis of the quantity of the reflected light. The measuring light beam is transmitted to the probe 12 from an external light source 14 through an optical fiber 13, for example. The reflected light is transmitted to a light measuring means 16 remote from the probe 12 by way of an optical fiber 13, for example. A reference light beam for determination of the optical density is transmitted to the light measuring means 16 by way of a separate optical fiber (not shown).

The tape 2 is stopped on the temperature maintaining means 11 where the sample liquid is deposited thereon and incubated at a desired temperature for a predetermined time. Thereafter, the reflection density of the corresponding analyzing unit is measured by the probe 12 and the light measuring means 16. In the case of the reaction rate analysis, the measurement is repeated more than twice. The sample liquid depositing means 9 is movable and it is moved away from the reflection density measuring section and the upper surface of the tape 2 is covered with an evaporation preventing cover 10.

The tape 2 is conveyed by a roller 17 which is intermittently driven by a driving means (not shown). Reference numeral 18 denotes an auxiliary roller 18 for winding the tape 2 around the roller 17. The tape 2 is received in a container 20 after passing the roller 17.

The evaporation preventing cover 10 is pivoted at one end thereof to be movable between an operative position in which it covers the tape 2 and a retracted position away from the reflection density measuring section, and before the tape 2 is conveyed, the cover 10 is moved to the retracted position.

## Claims

1. A method for quantification of a specific component, deposited on a chemical analysis tape (103), which includes a liquid spreading layer for spreading the sample liquid deposited thereon in a predetermined pitch,
   **characterized in that**,
   the width of the tape satisfies formula $\sqrt{2S/3P\pi}$ < W < $2\sqrt{S/P\pi}$ wherein S represents the volume of the sample liquid to be deposited on the tape, P represents the volume of the sample liquid which the spreading layer can receive per unit area ($mm^2$) thereof, and W represents the width of the tape in mm.

## Patentansprüche

1. Verfahren zur Quantifizierung einer spezifischen Komponente, welche auf einem chemischen Analysenband (103) aufgebracht ist, und welches eine Flüssigkeitsverteilschicht aufweist, um die Flüssigkeit, die darauf angeordnet ist, in einem vorbestimmten Abstand zu verteilen,
   **dadurch gekennzeichnet,**
   daß die Breite des Bandes die Formel $\sqrt{2S/3P\pi}$ < W < $2\sqrt{S/P\pi}$ erfüllt, wobei S das Volumen der Probe ist, welche auf dem Band aufzubringen ist, P das Volumen der Probenflüssigkeit, welches die Verteilschicht pro Flächeneinheit ($mm^2$) aufnehmen kann, und W die Breite des Bandes in mm bezeichnet.

## Revendications

1. Procédé de dosage quantitatif d'un composant spécifique déposé sur une bande d'analyse chimique (103), laquelle comprend une couche d'étalement de liquides destinée à l'étalement du liquide échantillon déposé sur celle-ci selon un écartement prédéterminé, caractérisé en ce que la largeur de la bande satisfait à la formule $\sqrt{2S/3P\pi}$ < W < $2\sqrt{S/P\pi}$ dans laquelle S représente le volume du liquide échantillon à déposer sur la bande, P représente le volume du liquide échantillon que peut recevoir la couche d'étalement par unité de surface ($mm^2$) et W représente la largeur de la bande en mm.

# F I G . 1

# F I G . 2

# F I G. 3

# F I G . 4

# F I G . 5